(19) Europäisches Patentamt
European Patent Office
Office européen des brevets

(11) Publication number: 0 076 037 B1

(12) EUROPEAN PATENT SPECIFICATION

(45) Date of publication of patent specification: 07.12.88

(51) Int. Cl.$^4$: C 12 N 15/00, C 12 P 21/00

(21) Application number: 82304516.6

(22) Date of filing: 26.08.82

# (54) Amplified expression of DNA sequences.

(30) Priority: 02.09.81 GB 8126584
17.03.82 GB 8207722

(43) Date of publication of application:
06.04.83 Bulletin 83/14

(45) Publication of the grant of the patent:
07.12.88 Bulletin 88/49

(84) Designated Contracting States:
AT BE CH DE FR GB IT LI LU NL SE

(56) References cited:
EP-A-0 034 470

CHEMICAL ABSTRACTS, vol. 94, no. 13, March 1981, p. 581, no. 101345n, Columbus, OH (US)

CHEMICAL ABSTRACTS, vol. 90, no. 5, 29th January 1979, p. 252, no. 36196r, Columbus, OH (US); I.G.YOUNG et al.: "Amplification of the respiratory NADH dehydrogenase of Escherichia coli by gene cloning"

(73) Proprietor: BIOGEN N.V.
15 Pietermaai
Willemstad Curacao, Netherlands Antilles (NL)

(72) Inventor: Kupper, Hans
41 Avenue Dumas
CH-1206 Geneva (CH)

(74) Representative: Bannerman, David Gardner et al
Withers & Rogers 4 Dyer's Buildings Holborn
London, EC1N 2JT (GB)

(56) References cited:
CHEMICAL ABSTRACTS, vol. 77, no. 5, 31st July 1972, p. 268, no. 31487h, Columbus, OH (US); D.B.CLEWELL et al.: "Effect of growth conditions on the formation of the relaxation complex of supercoiled Co1E1 deoxyribonucleic acid and protein in Escherichia coli"

ADVANCES IN APPLIED MICROBIOLOGY, vol. 25, 1979, pp. 57-74, Academic Press, Inc., (US); D.E.EVELEIGH et al.: "Increasing yields of extracellular enzymes"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 076 037 B1

(56) References cited:
JOURNAL OF BACTERIOLOGY, vol. 143, no. 1, July 1980, pp. 535-537; F.C.NEIDHARDT et al.: "Selective synthesis of plasmid-coded proteins by Escherichia coli during recovery from chloramphenicol treatment"

JOURNAL OF BACTERIOLOGY, vol. 110, no. 2, May 1972, pp. 667-676 (US); D.B.CLEWELL: "Nature of Co1E1 plasmid replication in Escherichia coli in the presence of chloramphenicol"

## Description

Technical field of the invention

This invention relates to the amplified expression of DNA sequences and to the improved production of amino acids, polypeptides and proteins coded for by those DNA sequences. More particularly, the invention relates to a process which amplifies the expression of multicopy plasmid-carried DNA sequences and improves the synthesis of multicopy plasmid-coded amino acids, polypeptides and proteins, affording high yields of those products, low contamination of those products by non-plasmid-coded products and ease of fermentation culture monitoring, handling and purification. The process of this invention is particularly useful in large scale fermentation processes where it affords the production of substantially higher yield of product per unit volume of culture than conventional processes.

Background art

Recent advances in molecular biology have made it possible to introduce the DNA coding for specific non-bacterial amino acids, polypeptides and proteins into a variety of host cells. In general, the DNA coding for the desired amino acid, polypeptide or protein product is introduced into an appropriate site in a cloning vehicle to form a recombinant DNA molecule. That molecule is then used to transform a compatible host and the host cultured to express the inserted DNA sequence and to produce the product coded for by that DNA sequence.

Several non-bacterial proteins have been produced in bacterial hosts using such recombinant DNA technology. These include, for example, leukocyte interferon (S. Nagata et al., "Synthesis in *E. coli* of A Polypeptide with Human Leukocyte Interferon Activity", *Nature*, 284, pp. 316—20 (1980)), antigens of human hepatitis B virus (C. J. Burrell et al., "Expression in *Escherichia coli* of Hepatitis B Virus DNA Sequences Cloned in Plasmid pBR322", *Nature*, 279, pp. 43—7 (1979) and M. Pasek et al., "Hepatitis B Virus Genes and their Expression in *E. coli*", *Nature*, 282, pp. 575—79 (1979)), SV40t antigen (T. M. Roberts et al., "Synthesis of Simian Virus 40t Antigen in *Escherichia coli*", *Proc Natl. Acad. Sci. USA*, 76, pp. 596—600 (1979)), and FMD viral antigens (H. Küpper et al., "Cloning of cDNA of Major Antigen of Foot and Mouth Disease Virus and Expression in *E. Coli*", *Nature*, 289, pp. 555—59 (1981)).

The level of production of a product in such host cells is governed by three major factors: the number of copies of the DNA sequence that codes for the product within the cell, the efficiency with which those DNA sequences are transcribed and the efficiency with which the resultant messenger RNA ("mRNA") is translated. Efficiency of transcription and translation (which together comprise expression) is in turn dependent upon the nucleotide sequences which are normally situated ahead of the sequences coding for the desired products. These nucleotide sequences or expression control sequences define, inter alia, the location at which RNA polymerase interacts (the promoter sequence) to initiate transcription and at which ribosomes bind and interact with the mRNA (the product of transcription) to initiate translation to the desired product.

A variety of expression control sequences are now available to improve the efficiency of expression (transcription and translation) of cloned DNA sequences. These expression control sequences are of two main types—constitutive expression control sequences and controllable expression control sequences. Constitutive expression control sequences, i.e., β-lac, lac-UV5 or tet, continuously function during host cell growth to express the cloned DNA sequence and to produce the product coded for by that DNA sequence. Controllable expression control sequences, i.e., trp, $\lambda P_L$, or $\lambda P_R$, may be regulated, i.e., switched on or off, during host cell growth so that expression of the cloned DNA sequence may be "switched on" at the most appropriate time in a culture's growth cycle.

Both types of expression control sequences have been used in processes to permit the expression of multicopy plasmid-carried DNA sequences and the production of products coded for by those sequences. The particular process employed depends in large measure on the type of expression control sequence. For constitutive expression control sequences, a culture of host cells transformed with the multicopy plasmid-carried DNA sequence coding for the desired product is grown in appropriate media and under appropriate conditions until the exponential growth phase has been substantially completed. The cells are then harvested and the desired product recovered and purified using well-known processes. Most often in large scale cultures, i.e. from 100 ml to many thousands of liters, an overnight culture of the transformed host is first prepared and that culture is then used to inoculate about a 100 times larger volume of culture media. The larger-volume culture then undergoes a lag phase—when the cells are readjusting to growth conditions and culture environment—of about 2 h before exponential cell growth begins. Again, however, the culture is harvested after the exponential growth phase is substantially completed and the desired product recovered and purified.

This process is beset by several disadvantages. First, the method is slow. Often 7—20 h of cell growth and careful monitoring of cell density are required before harvesting can begin. In addition, the amount of the desired product per unit of culture volume is usually relatively low because the host cell also produces all of the other products coded for by its plasmid and chromosomal DNAs. Finally, recovery and purification of the desired product is often difficult and expensive because of its low yield—necessitating the culturing and handling of large culture volumes to recover useful amounts—and because of its contamination with so many other similar proteins and related products.

For controllable expression control sequences, a culture of host cells transformed with the multicopy plasmid-carried DNA sequence coding for the desired product is grown in media and under conditions where the expression control sequence is "switched off". During growth the concentration of cells is monitored, usually by the optical density of the culture. When the density reaches a pre-determined point (usually about the mid-point of the exponential growth phase), the expression control sequence is "switched on" by addition of an activating substance like tryptophan or by a temperature shift. The culture of host cells now expressing the inserted DNA sequence is then grown to stationary phase before harvesting and subsequent recovery and purification of the desired product. Again, in large scale cultures, an overnight culture is employed as an inoculum for about a 100 times larger volume culture. Again, the culture undergoes a lag phase of several hours before exponential growth begins and the cell density approaches the point chosen for activation and continued growth before harvesting.

This culture method is also beset by several disadvantages. Again, the method is slow. Often 6—10 h are required before harvesting can begin. The method also requires careful monitoring of the cell density and rapid activation of the culture at the appropriate density "to switch on" expression of the inserted DNA sequence. In addition, while the relative amount of desired product synthesized by the cell after "switch on" is generally high as compared to the amounts of other products being made at that time by the cell, the total amount of desired product per unit of culture volume is still relatively low because the host cell produces the products coded for by its chromosomal DNA before switch on, since those are not under the control of the regulated expression control sequence, and because after "switch on" the host cell continues to produce the products coded for by its chromosomal DNA and also produces any other proteins coded for by the plasmid DNA that are under the control of the "switched on" expression control sequence. Finally, recovery and purification of the desired protein are difficult and expensive because of its relatively low yield—necessitating the culturing and handling of large culture volumes to recover useful amounts—and because of its contamination with other similar proteins and related products.

A variety of means to increase the number of copies per cell of a DNA sequence coding for a desired product is also available. The most usual means is to use a multicopy plasmid for cloning the DNA sequence coding for a desired product. When such multicopy plasmid is transformed into a host cell, it replicates in the cell so that each cell contains 20—50 copies of the plasmid. Accordingly, each cell also contains 20—50 copies of the DNA sequence that codes for the desired product since that sequence is carried on the plasmid. The extra copies of the DNA sequence are thus available for expression and production of the desired product and accordingly afford a higher yield of that product per cell.

Other techniques have allowed the copy number of a multicopy plasmid to be increased well beyond the usual 20—50 copies per cell. These techniques include, for example, treatment of cells transformed with a multicopy plasmid-carried DNA sequence coding for a desired product with chloramphenicol and growing of cells transformed with a multicopy plasmid-carried DNA sequence coding for a desired product under conditions of amino acid starvation.

Chloramphenicol treatment of cells and growth of cells under conditions of amino acid starvation affect the ability of the cells to make proteins. While the lack of some of these proteins prevents replication of the cell and its chromosomal DNA, their absence does not initially prevent the continued replication of plasmid-carried DNA. Accordingly, the number of copies per cell of the multicopy plasmid-carried DNA increases. For example, after chloramphenicol treatment it has been estimated that the host cell may contain up to about 2000 copies of the plasmid and stationary phase cultures (a condition of amino acid starvation) have been estimated to contain about 400—500 copies per cell of the plasmid.

However, chloramphenicol treatment, as described by F. G. Neidhardt et al., "Selective Synthesis of Plasmid-Coded Proteins by *Escherichia coli* During Recovery from Chloramphenicol Treatment", *J. Bacteriol.*, 143, pp. 535—37 (1980), is very cumbersome—a culture in the exponential growth phase must be treated with chloramphenicol overnight, the cells collected by centrifugation, the chloramphenicol washed out and the cells resuspended in media. Accordingly, it has only been employed with very small scale (5 ml) cultures where collecting the cells, washing them and resuspending them is not a difficult procedure (Neidhardt et al., *supra*). The method has, in fact, more usually been employed to produce large numbers of plasmids and quantities of plasmid-carried DNA for isolation and analysis since less cumbersome collecting of cells, washing and resuspension is required to recover the amplified plasmids.

Amino acid starvation has also been occasionally employed to permit the recovery of large amounts of plasmid-coded DNA. However, it has not been used as a means for increasing the production or yield of products coded for by plasmid-carried DNA sequences. In fact, conventional fermentation processes serve to dissipate any high copy number of plasmid-carried DNA of a stationary phase culture by first diluting the culture into a larger media volume and then allowing growth to proceed through the exponential phase (at which time the copy number is back to its original level—20 to 50 copies per cell) before harvesting.

Therefore, the promise of recombinant DNA technology to produce useful amounts of various products has been delayed by the disadvantages inherent in the various techniques presently available to express plasmid-carried DNA sequences an to produce the products coded for by those sequences.

## Disclosure of the invention

The present invention generally solves the problems referred to above by providing a method to amplify the expression of multicopy plasmid-carried DNA sequences and to increase the production of amino acids, polypeptides and proteins coded for by those DNA sequences. More particularly, the process of this invention improves the synthesis of products coded for by multicopy plasmid-carried DNA sequences affording high yields of the products, low contamination of the products by products of non-plasmid-carried DNA sequences and ease of culture monitoring, handling and purification.

As will be appreciated from the disclosure to follow the process of this invention comprises the steps of increasing thy copy number per cell of a multicopy plasmid-carried DNA sequence coding for a desired product; combining culture media or other nutrients with a culture of said increased copy number cells, the volume of the culture media being preferably between 0.05 to 10 times the volume of the culture and most preferably between 0.05 and 0.2 times that volume; culturing the resultant culture under conditions appropriate for expression of the multicopy plasmid-carried DNA sequence; and harvesting the resulting culture before substantial exponential growth has occurred i.e. preferably about 2—3 h after the culture and media were combined.

Brief description of the drawings

Figure 1 is a pictorial representation of plasmid pPL VP1.

Figure 2 is a graphical presentation of cell density ($OD_{650}$) vs. time (h) for a culture of *E. coli* M5219 (pPL VP1) using conventional processes.

Figure 3 is a graphical presentation of cell density ($OD_{650}$) vs. time (h) for a culture of *E. coli* M5219 (pPL VP1) using the process of this invention.

Figure 4 is a pictorial representation of plasmid pHBc R-11.

Figure 5 is a graphical presentation of cell density ($OD_{650}$) vs. time (h) for a culture of *E. coli* HB101 (pHBc R-11) using conventional processes.

Figure 6 is a graphical presentation of cell density ($OD_{650}$) vs. time (h) for a culture of *E. coli* HB101 (pHBc R-11) using the process of this invention.

Best mode of carrying out the invention

In order that the invention herein described may be more fully understood, the following detailed description is set forth. In this description the term "host organism" is used broadly. For example, it includes both gram negative and gram positive bacterial hosts such as strains of *E. coli*, e.g., *E. coli* HB101, *E. coli* X1776, *E. coli* X2882, *E. coli* DS410, *E. coli* C600, *E. coli* MRCl, *E. coli* M5219 and strains of *Pseudomonas, Bacillus subtilis, Bacillus steareothermophilus* and other *bacilli* or yeasts and other fungi. Preferably, *E. coli* C600 is used. The selection of a particular host is not part of this invention. Rather, the invention is applicable generally to the amplified expression of multicopy plasmid-carried DNA sequences which have been employed to transform those host organisms and to the production of the products coded for by those sequences in such hosts.

In addition, the process of this invention is applicable to a wide variety of proteins, polypeptides, amino acids and other products. For example, products such as the active components of vaccines or other pharmaceutically active products, agriculturally or other commercially useful compositions, enzymes, amino acids, industrial chemicals, antibiotics, food stuffs, and the like are usefully produced by the process of this invention. Again, the selection of a particular product is not part of this invention. Rather, the invention is applicable generally to the amplified expression of multicopy plasmid-carried DNA sequences coding for such products and to the production of such products in hosts transformed with those multicopy plasmid-carried DNA sequences.

Finally, the numerous techniques and processes for growing or fermenting cultures of host organisms or for the recovery or purification of products from them are not part of this invention. Rather, this invention is a useful complement to those techniques and improves the expression of multicopy plasmid-carried DNA sequences and the production of desired products from them.

It should, of course, be understood that not every host organism or every growth, recovery or purification technique may be usefully employed to produce a specific product in a particular host organism by the processes of this invention. Rather, those of skill in the art should consider various factors known in the art to select a preferred host and preferred conditions for the production and purification of a desired product.

For example, a wide variety of host/plasmid-cloning vehicle combinations have been employed in recombinant DNA technology to prepare transformed host organisms that produce a desired foreign product. For example, useful multicopy plasmid-cloning vehicles may consist of various known bacterial plasmids, e.g., multicopy plasmids from *E. coli* incuding pMB9, pBR325, pBR322 and their derivatives; wider host range multicopy plasmids, and cloning vehicles derived from combinations of multicopy plasmids and phage DNAs such as plasmids which have been modified to employ phage DNA or other expression control sequences or multicopy yeast plasmids such as 2 μ plasmids or derivatives thereof. Various DNA sequences may be cloned in those multicopy plasmid-cloning vehicles at well-recognized sites in them to produce hybrid or recombinant DNA molecules. These molecules are useful, either as produced or after appropriate modification to improve their expression characteristics—preferably to place the DNA sequence coding for a desired product under the control of a strong expression control sequence so that such DNA sequence will be expressed more effectively than other DNA sequences carried by the plasmid—or to modify the product actually expressed from them, to transform host organisms, as described above, and to produce the products coded for by the inserted DNA sequences or portions thereof.

Illustrative of the foreign products that have been produced by such transformed host organisms are hepatitis B viral antigens, foot and mouth disease viral antigens, leukocyte interferon, fibroblast interferon, the A and B chains of human insulin, rat growth hormone, mouse dihydrofolate reductase, somatostatin, human growth hormone and the like.

Within the above general technology, the present invention is characterized by a simple and efficient process for amplifying the expression of multicopy plasmid-carried DNA sequences and for producing the amino acids, polypeptides and proteins coded for by those DNA sequences in improved yields and with less contamination by other proteins. The process is also characterized by ease of handling and culturing the host cell cultures that produce such products and by the ease of recovery and purifying such products from the culture. The process makes use of the lag phase in a culture's growth cycle to produce large amounts of the desired products while at the same time substantially avoiding the production of undesired products.

The process comprises the steps of increasing the copy number per cell of a multicopy plasmid-carried DNA sequence encoding a desired product; combining a culture medium with a culture of said increased copy number cells, culturing the resultant culture under conditions appropriate for expression of said DNA sequence; and harvesting the resulting culture before substantial exponential cell growth occurs.

While not wishing to be bound by theory, it is believed that the amplified expression of multicopy plasmid-carried DNA sequences and the improvement in the production of products coded for by those sequences results from the process of this invention because during lag phase the host cells are not replicating but rather adjusting to the new environmental conditions by preferential DNA and protein synthesis. Because few new cells are made, the copy number per cell of the plasmid-carried DNA remains very high related to the amount of chromosomal DNA. Therefore, most of the products synthesized by the host cells during the lag phase are those products that are coded for by plasmid-carried DNA sequences. It is also believed that the unexpected results of the process of this invention are dependent on harvesting the culture before substantial exponential growth occurs, because the copy number per cell of the multicopy plasmid-carried DNA sequences relative to chromsomal DNA is readjusted to the original level as exponential growth begins. Accordingly, the products synthesized by the cell during its exponential growth phase are a diverse mixture of chromosomal and plasmid-coded products, with the latter being present in relatively small amount.

We prefer to increase the copy number per cell by growing a culture of cells that have been transformed with a multicopy plasmid-carried DNA sequence coding for a desired product overnight and more preferably, in the particular case of strains of *E. coli*, for 18 hours or more. This period of growth results in a dense stationary phase culture. It also results in an estimated copy number per cell of a plasmid-carried DNA sequence of between about 400 and 500 for a multicopy plasmid like pBR322.

Although conventional media may be used in preparing the above-described stationary phase culture, very minimal media may also be used since only DNA synthesis, not protein synthesis, is required for the overnight culture. Moreover, the media employed may be usefully adjusted with various known additives that favor the DNA synthesis, e.g., glycerol and other carbon sources. In any case, however, the media should be buffered, for example, by adding potassium phosphate, to avoid acidic conditions in the the stationary phase. The temperature and other conditions used to prepare the above-described stationary phase culture should be those conventionally used to grow the host cells of the cultures. However, for those host cells that have been transformed with multicopy plasmid-carried DNA sequences under the control of controllable expression control sequences, the growth conditions are preferably ones that limit or avoid expression of that DNA sequence. For example, transformed cells which are activated to expression by a temperature shift from 28°—30°C to 42°—44°C should be grown at 28°—30°C to prepare the stationary phase culture.

After preparation of the high copy-number culture, it is combined in accordance with the process of this invention with a volume of culture media. Although the ratio of the volume of the culture to the volume of the culture media may vary widely, enough media must be added to assure regrowth of the cells to exponential phase. It is therefore preferable to strike a balance between the desire to use as little media as possible to reduce the costs of the process and the size of the equipment necessary to handle, to treat and to purify large culture volumes and the need to use enough media to allow the cells to grow and to carry out protein synthesis. Which this selection depends to some extent as the host, usually, the volume of the added culture media is 0.05 to 10 times the volume of the culture and more preferably 0.05 to 0.2 times that volume are employed. It should of course be understood that while we have described this step in our process as the combination of a culture with a culture media, the culture media could equally well be added to the culture. In large scale fermentation, that latter method is preferably chosen.

The media used for the large volume culture may be conventional media useful for the growth of the host cell organism. Preferably, the media may also be enriched with amino acids since optional protein synthesis is aimed for in the resulting culture. The temperature and other conditions of the culture and growth should be those conventionally used to grow the host cell of the culture. However, for those host cells that have been transformed with plasmid-carried DNA sequences under the control of controllable expression control sequences, the conditions of the media (before combination with the culture) should be ones that .activate the expression of the DNA sequence. For example, cells which are activated to expression by a temperature shift from 28°—30°C to 42°—44°C should be combined with a culture media already at 42°—44°C, because immediate expression of those DNA sequences is preferable. Such technique, of course, also avoids the need to monitor the growth of the culture and to shift rapidly the growth conditions of a growing culture for "switch on" of expression as is required in conventional fermentation of hosts containing DNA sequences under the control of controllable expression control sequences.

The time the diluted culture is permitted to grow before harvesting may also vary widely according to the particular host, plasmid, conditions and media. It is only necessary to maximize the advantages of this invention that the cells be harvested at a time when they have produced a relatively large amount of the desired product as compared to the other cellular products. Preferably, the cells are harvested before substantial exponential growth has occurred and, more preferably, in the particular case of *E. coli* strains, after about 3 h of growth.

In order that this invention may be more fully understood, the following examples of the process of this invention are set forth. These examples are for purposes of illustration only and this invention should not be considered to be limited by any recitation used herein.

Example 1

Example 1 is a comparison of the expression of a plasmid-carried DNA sequence coding for a polypeptide displaying the antigenicity of the VP1 protein of foot and mouth disease virus type $O_1K$ and the production of that polypeptide using a conventional process and using one embodiment of the process of this invention.

In this example, plasmid pPL VP1 was constructed from plasmid pPLc24 and plasmid pFMDV-1034 as described in Küpper et al., *supra*. The plasmid has a DNA sequence coding for a polypeptide displaying the antigenicity of the VP1 protein of FMDV, type $O_1K$, inserted in it under the control of the $P_L$ controllable expression control sequence (Figure 1). This expression control sequence is "switched off" at about 28°C and "switched on" at about 43°C. Plasmid pPL VP1 was used to transform *E. coli* M5219 (K12 M72 $lac_{am}trp_{am}Sm^R$ (λcI857 ΔHlbio252) (H. Greer, "The *Kil* Gene of Bacteriophage λ", *Virology*, 66. pp. 589—604 (1975)).

An overnight culture of *E. coli* M5219, transformed with plamsid pPL VP1, was prepared in L Broth containing 40 μg/ml amplicillin (the transformed host is resistant to ampicillin (Figure 1)) at 28°C.

Four ml of the above-described overnight culture were added to 400 ml L Broth (1/100 dilution) and the culture allowed to grow at 28°C, the $OD_{650}$ being monitored during culture growth. When the $OD_{650}$=0.9 (~3.5 h), the temperature of the culture was raised to 43°C. After 3 h at 43°C the culture was harvested by centrifugation at 10000 rpm for 10 min (Figure 2). The resulting pellet was taken up in 10 ml 1×PBS (phosphate buffered saline) and aliquots removed for analysis. Urea to 5 M and SDS to 1% were added to each aliquot. The resulting aliquot solutions were boiled for 5 min and the supernatants recovered by centrifugation for 20 min at 15000 rpm. The amount of polypeptide displaying the antigenicity of VP1 in the supernatant of each aliquot was determined by SDS-gel electrophoresis and complement fixation assay.

One hundred ml of the above-described overnight culture were also added to 400 ml of L Broth preheated to 43°C (1/4 dilution) and the culture allowed to grow at 43°C for 3 h (Figure 3). The cells were harvested and the supernatant removed and analyzed as before.

The results of the two processes are described below:

| | Conventional process | Process of this invention |
|---|---|---|
| Time (h) | 6.5 | 3 |
| Desired protein (mg/l) (estimated by gel electrophoresis) | 3—6 | 15—30 |
| Desired protein (mg/l) (estimated by complement fixation assay) | 3—6 | 15—30 |

Therefore, using this embodiment of the process of this invention affords 3 to 8 times higher yields per unit volume of the desired product. Moreover, it is plain that because of these higher yields the purification and recovery of the desired product is much easier and more efficient since 3 to 8 times less culture volume must be used and processed and 3 to 8 times less impurities must be removed to isolate the same amount of product. In addition, where, as here, multicopy plasmid-carried DNA sequences under the control of controllable expression control sequences are employed, the culture need not be monitored and then quickly shifted during growth to "switch on" the expression of the desired DNA sequence.

Example 2

Example 2 is a comparison of the expression of the DNA sequence of pPL VP1 and the production of a VP1-related product in another host using a conventional process and the process of this invention.

Plasmid pPL VP1, described above and depicted in Figure 1, was employed to transform *E. coli* C600 (pCI$t_{ts}$857) (a C600 strain carrying the repressor on a plasmid). An overnight culture of this transformed host was prepared at 28°C.

As before 4 ml of the overnight culture were added to 400 ml L Broth (1/100 dilution) and the culture allowed to grow at 28°C, the $OD_{650}$ being monitored during culture growth. When the $OD_{650}=0.9$ (~3.5 h), the temperature of the culture was raised to 43°C. After 3 h at 43°C the culture was harvested as before and the supernatant recovered and analyzed as before.

In addition, 100 ml of the overnight culture were added to 400 ml L Broth (1/4 dilution), preheated to 43°C, and the culture allowed to grow for 3 h. After harvesting and recovery, as before, the supernatant was analyzed for polypeptides displaying the antigenicity of VP1.

The results of the two processes are described below:

| | Conventional process | Process of this invention |
|---|---|---|
| Time (h) | 6.5 | 3 |
| Desired protein (mg/l) (estimated by gel electrophoresis) | 6—12 | 30—60 |
| Desired protein (mg/l) (estimated by complement fixation assay) | 6—12 | 30—60 |

Therefore, using this embodiment of the process of this invention with a different host strain that had been transformed with pPL VP1 afforded similarly improved results as compared to conventional processes (3 to 8 times higher yield per unit volume of desired product).

Example 3

Example 3 is another fermentation using the process of this invention for the expression of the DNA sequence of pPL VP1 and the production of a VP1-related product in *E. coli* C600 (pCI$_{ts}$857).

Plasmid pPL VP1, described above and depicted in Figure 1, was employed to transform *E. coli* C600 (pCI$_{ts}$857) as in Example 2. An overnight culture of this transformed host was prepared at 28°C.

To prepare an inoculum 0.1 ml of that culture were added to 5 ml L broth, supplemented with kanamycin (30 µg/ml) and ampicillin (40 µg/ml), and the culture grown overnight at 30°C. 0.1 ml of this culture were then added to 10 ml L broth, supplemented as before, and grown for 8 h at 30°C. 100 ml of L broth, supplemented as before, were then added and growth continued overnight at 30°C.

100 ml of the above-described overnight culture were added to 900 ml culture medium, containing $K_2HPO_4$ (4 g/l), $KH_2PO_4$ (1 g/l), $NH_4Cl$ (1 g/l), $CaCl_2 \cdot 6H_2O$ (0.01 g/l), $K_2SO_4$ (2.6 g/l), 1M $MgCl_2$ (2 ml/l), trace elements (10 ml/l), casamino acids (20 g/l), yeast extract (3 g/l), 87% glycerol (46 g/l), ampicillin (40 µg/l) and kanamycin (30 µg/l), that had been presterilized at 121°C for 15 min. The culture was grown at 30°C for 24 h (pH=7 (controlled by 25% $NH_4OH$ and 25% $H_3PO_4$), $O_2>30\%$). After 24 h the O.D.$_{650}$ was about 33.

The temperature of the culture was then raised to 42°C and 100 ml of medium, containing 22 g casamino acids, 3.3 g yeast extract and 25.3 g 87% glycerol and sterilized as before, were added (1/0.1 dilution). The culture was held at 42°C for 2 h (pH control as before, O.D.$_{650}\cong42.4$) and harvested.

After recovery, as before, the supernatant was analyzed for polypeptides displaying the antigenicity of VP1: ~20% of total cell proteins; about 1600 mg/l (estimated by gel electrophoresis).

Therefore, using this embodiment of the process of this invention (dilution 1/0.1) afforded 125 to 250 times the yield per unit volume of conventional processes.

Example 4

Example 4 is a comparison of the expression of a plasmid-carried DNA sequence coding for a polypeptide displaying the antigenicity of hepatitis B virus core antigen ("HBcAg") and the production of that polypeptide using a conventional process and using the process of this invention.

In this example, plasmid pHBc R-11 was employed. Plasmid pHBc R-11 (Figure 4) contains the DNA sequence coding for a 140 amino acid-containing polypeptide that displays the antigenicity of hepatitis B virus core antigen. As shown in Figure 4 the HBcAg-related DNA sequence in pHBc R-11 is under the control of a constitutive expression control sequence—Lac-UV5. Plasmid pHBc R-11 was employed to transform *E. coli* HB101.

An overnight culture of *E. coli* HB101 (pHBc R-11) was prepared in L Broth containing 40 µg/ml ampicillin (the transformed host is resistant to ampicillin (Figure 4)) at 37°C.

Four ml of the above-described overnight culture were added to 400 ml L Broth (1/100 dilution) and the culture allowed to grow at 37°C until the end of the exponential growth phase (~6—7 h) (Figure 5). The cells were harvested by centrifugation at 10000 rpm for 10 min. The pellet was redissolved in 10 ml 1×PBS and aliquots of that solution selected and NP40 added to 0.5%. The aliquots were sonicated 3 times for 1 min and centrifuged at 10000 rpm for 10 min. The amount of product displaying the antigenicity of HBcAg in each aliquot was then determined by complement fixation assay.

One hundred ml of the overnight culture were also added to 400 ml of L Broth at 37°C (1:4 dilution). The culture was grown for 3 h at 37°C (Figure 6) and the cells harvested as before. Aliquots prepared as before were then analyzed for the amount of product displaying HBcAg antigenicity.

The results of the two processes are described below:

| | Conventional process | Process of this invention |
|---|---|---|
| Time (h) | 6—7 | 3 |
| Desired protein (mg/l) (estimated by complement fixation assay) | 1—2 | 8—16 |

Therefore, using this embodiment of the process of this invention afforded about 8 times higher yields per unit volume of the desired product. Moreover, it is plain that because of these higher yields the purification and recovery of the desired product is much easier and more efficient since 8 times less culture volume must be used and processed and 8 times less impurities must be removed to isolate the same amount of product.

While we have hereinbefore presented a number of embodiments of this invention, it is apparent that our basic process may be altered to provide other embodiments which utilize it. Therefore, it will be appreciated that the scope of this invention is to be defined by the claims appended hereto rather than the specific embodiments which have been presented hereinbefore by way of example.

**Claims**

1. A process for amplifying the expression of multicopy plasmid-carried DNA sequences and improving the production of the products coded for by those DNA sequences comprising the steps of increasing the copy number per cell of a multicopy plasmid-carried DNA sequence coding for a desired product by culturing the cells transformed by the multicopy plasmid until exponential growth has substantially ceased; combining culture media with a culture of said increased copy number cells, said culture media having 0.05 to 10 times the volume of the culture of said increased copy number cells; and culturing the resultant culture under conditions appropriate for expression of the multicopy plasmid-carried DNA sequence.

2. The process of claim 1, wherein the culture media has 0.05 to 0.2 times the volume of the culture of said increased copy number cells.

3. The process of claim 1, wherein the culture media has 3 to 5 times the volume of the culture of said increased copy number cells.

4. The process of any one of claims 1 to 3, wherein the cell is selected from strains of *E. coli, Bacillus, Pseudomonas,* yeasts and other fungi.

5. The process of claim 4, wherein the cell is *E. coli* C600.

6. The process of any one of claims 1 to 5, wherein said DNA sequence is selected from DNA sequences coding for active components of vaccines, pharmaceutical products, agricultural products, enzymes, hormones, polypeptides, proteins, amino acids, industrial chemicals, antibiotics and food stuffs.

7. The process of claim 6, wherein said DNA sequence is selected from DNA sequence coding for products displaying an immunological or biological activity of leukocyte interferon, products displaying an immunological or biological activity of fibroblast interferon, products displaying the antigenicity of FMD viral antigens, products displaying the biological activity of human or animal growth hormones, products displaying the biological activity of human insulin and products displaying the antigenicity of hepatitis B viral antigens.

**Patentansprüche**

1. Verfahren zur Amplifikation der Expression von in Multicopy-Plasmiden enthaltenen DNA-Sequenzen und zur Verbesserung der Produktion der von diesen DNA-Sequenzen codierten Produkte, wobei man pro Zelle die Kopienzahl einer in einem Multicopy-Plasmid enthaltenen DNA-Sequenz, die für eine gewünschtes Produkt codiert, erhöht durch Züchtung der von dem Multicopy-Plasmid transformierten Zellen bis die exponentielle Wachstumsphase praktisch aufhört; Kulturmedien mit einer Kultur der Zellen mit erhöhter Kopienzahl vereinigt, wobei die Kulturemedien das 0,05- bis 10fache Volumen der Kultur der Zellen mit erhöhter Kopienzahl aufweisen; und die erhaltene Kultur unter Bedingungen züchtet, die zur Expression der im Multicopy-Plasmid enthaltenen DNA-Sequenz geeignet sind.

2. Verfahren nach Anspruch 1, wobei die Kulturmedien das 0,05- bis 0,2fache Volumen der Kultur der Zellen mit erhöhter Kopienzahl aufweisen.

3. Verfahren nach Anspruch 1, wobei die Kulturmedien das 3- bis 5fache Volumen der Kultur der Zellen mit erhöhter Kopienzahl aufweisen.

4. Verfahren nach einem der Ansprüche 1 bis 3, wobei die Zelle aus Stämmen von *E. coli, Bacillus, Pseudomonas*, Hefen und anderen Pilzen ausgewählt ist.

5. Verfahren nach Anspruch 4, wobei die Zelle *E. coli* C600 ist.

6. Verfahren nach einem der Ansprüche 1 bis 5, wobei die DNA-Sequenz ausgewählt ist aus DNA-Sequenzen, die für aktive Bestandteile von Impfstoffen, Arzneistoffen, landwirtschaftlichen Produkten, Enzymen, Hormonen, Polypeptiden, Proteinen, Aminosäuren, Industrie-Chemikalien, Antibiotika und Nahrungsmitteln codieren.

7. Verfahren nach Anspruch 6, wobei die DNA-Sequenz ausgewählt ist aus DNA-Sequenzen, die für Produkte mit immunologischer oder biologischer Aktivität von Leukozyten-Interferon, Produkte mit immunologischer oder biologischer Aktivität von Fibroblasten-Interferon, Produkte mit der Antigenität von Maul- und Klauenseuche-Virus-Antigenen, Produkte mit der biologischen Aktivität von menschlichen oder tierischen Wachstumshormonen, Produkte mit der biologischen Aktivität von menschlichem Insulin oder für Produkte mit der Antigenität von Hepatitis B-Virus-Antigenen codieren.

**Revendications**

1. Un procédé pour amplifier l'expression de séquence d'ADN porté par le plasmide multicopies, et pour améliorer la production de produits codés pour, par ces séquences d'ADN, qui comprend les étapes:

d'augmenter le nombre de copies par cellule d'une séquence d'ADN porté par le plasmide multicopies, codant pour le produit désiré en effectuant la culture des cellules transformées par le plasmide multicopies jusqu'à ce que la pousse exponentielle ait cessé essentiellement;

de combiner des milieux de culture avec une culture desdites cellules dont le nombre de copies est augmenté, lesdits milieux de culture ayant 0,05 à 10 fois le volume de la culture desdites cellules dont le nombre de copies est augmentée;

et de cultiver la culture résultante dans des conditions appropriées pour l'expression de la séquence d'ADN porté par le plasmide multicopies.

2. Le procédé de la revendication 1 dans lequel le milieu de culture à 0,05 à 0,2 fois le volume de la culture desdites cellules dont le nombre de copies est augmenté.

3. Le procédé de la revendication 1 dans lequel le milieu de culture à 3 à 5 fois le volume de la culture desdites cellules dont le nombre de copies est augmenté.

4. Le procédé selon l'une des revendications 1 à 3 dans lequel est choisi parmi les souches d'*E. Coli*, Bacillus, Pseudomonas, les levures et autres champignons.

5. Le procédé de la revendication 4 dans lequel la cellule est celle d'*E. coli* C600.

6. Le procédé selon l'une des revendications 1 à 5 dans lequel ladite séquence d'ADN est choisie parmi les séquences d'ADN codant pour les composants actifs de vaccins, de produits pharmaceutiques, de produits de l'agriculture, des enzymes, des hormones, des polypeptides, des protéines, des aminoacides, des substances chimiques industrielles, des antibiotiques et des dérivés alimentaires.

7. Le procédé de la revendication 6, dans lequel ladite séquence d'ADN est choisie parmi les séquences d'ADN codant pour de substances manifestant une activité immologique ou biologique de l'interféron de leucocyte, les substances manifestant une activité immunologique ou biologique de l'interféron de fibroblaste, les substances manifestent le pouvoir antigénique des antigènes viraux du Foot and Mouth disease (FMD), les substances manifestant l'activité biologique des hormones de croissance humaines ou animales, les substances manifestant l'activité biologique de l'insuline humaine et les substances manifestant le pouvoir antigénique des antigènes du virus de l'hépatite B.

FIG. 1

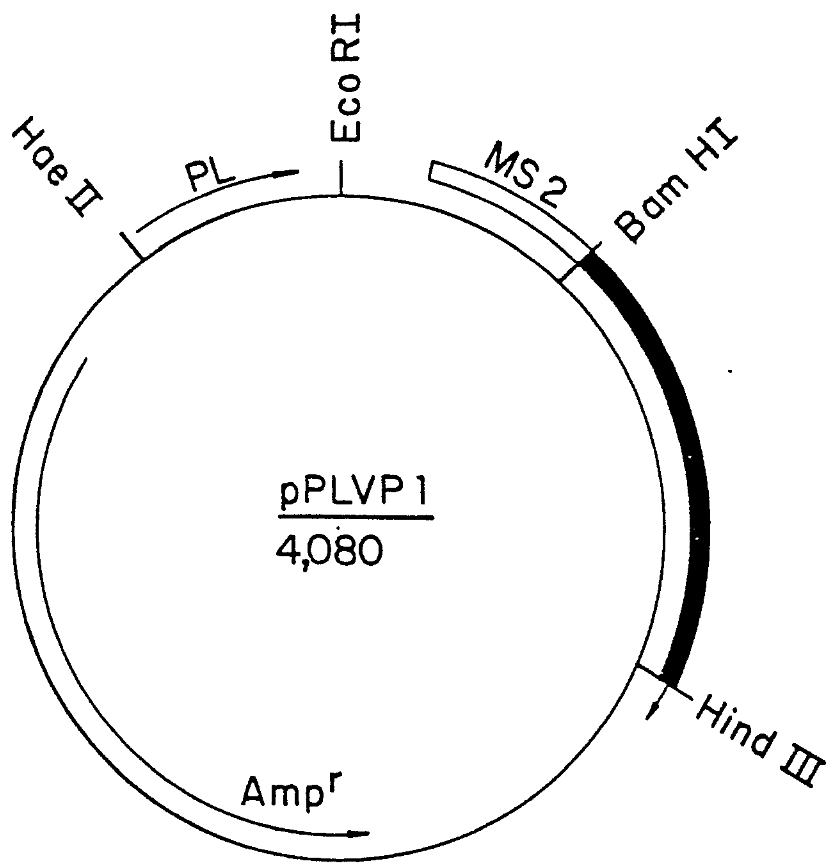

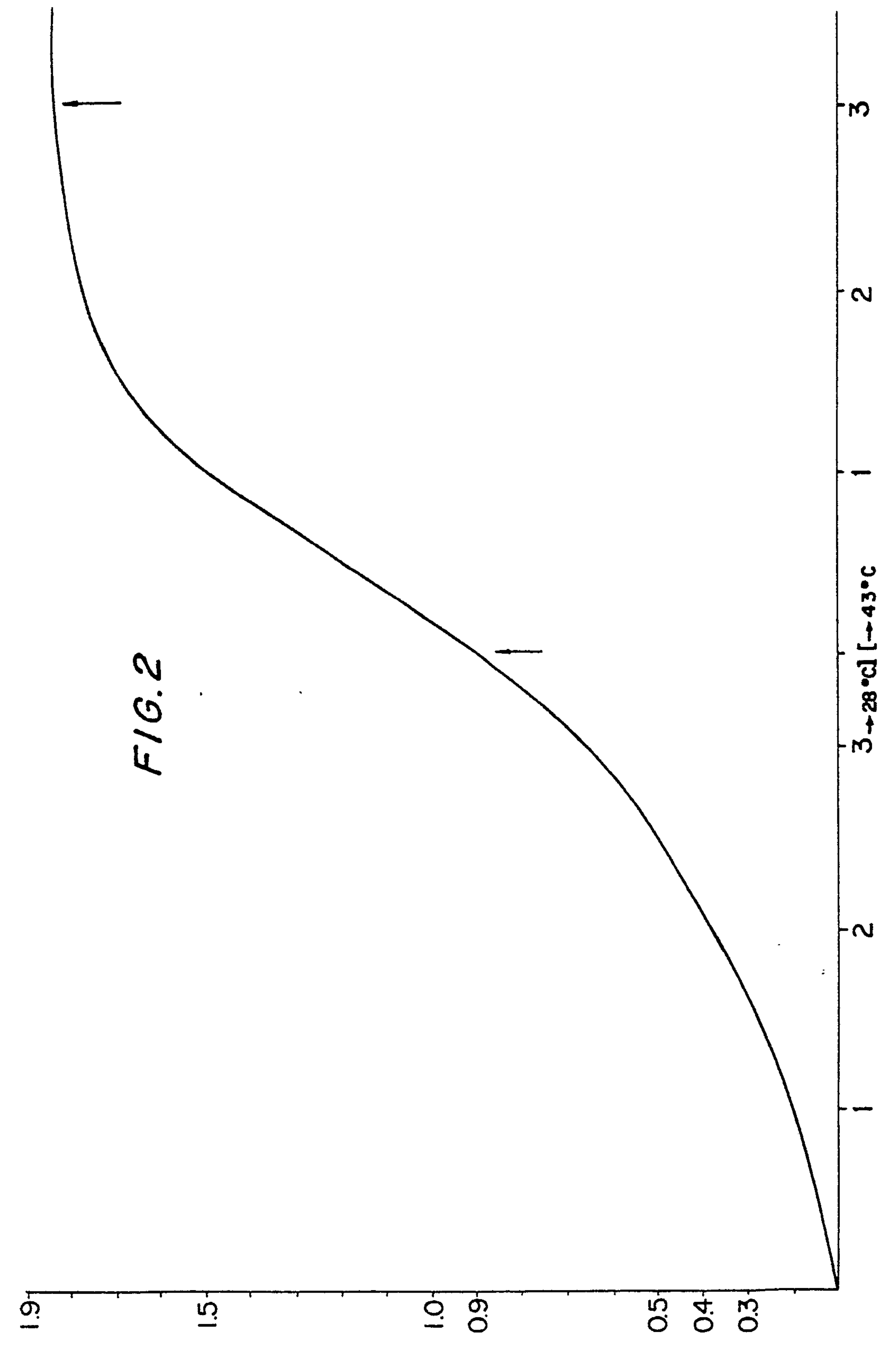


FIG. 2

FIG.3

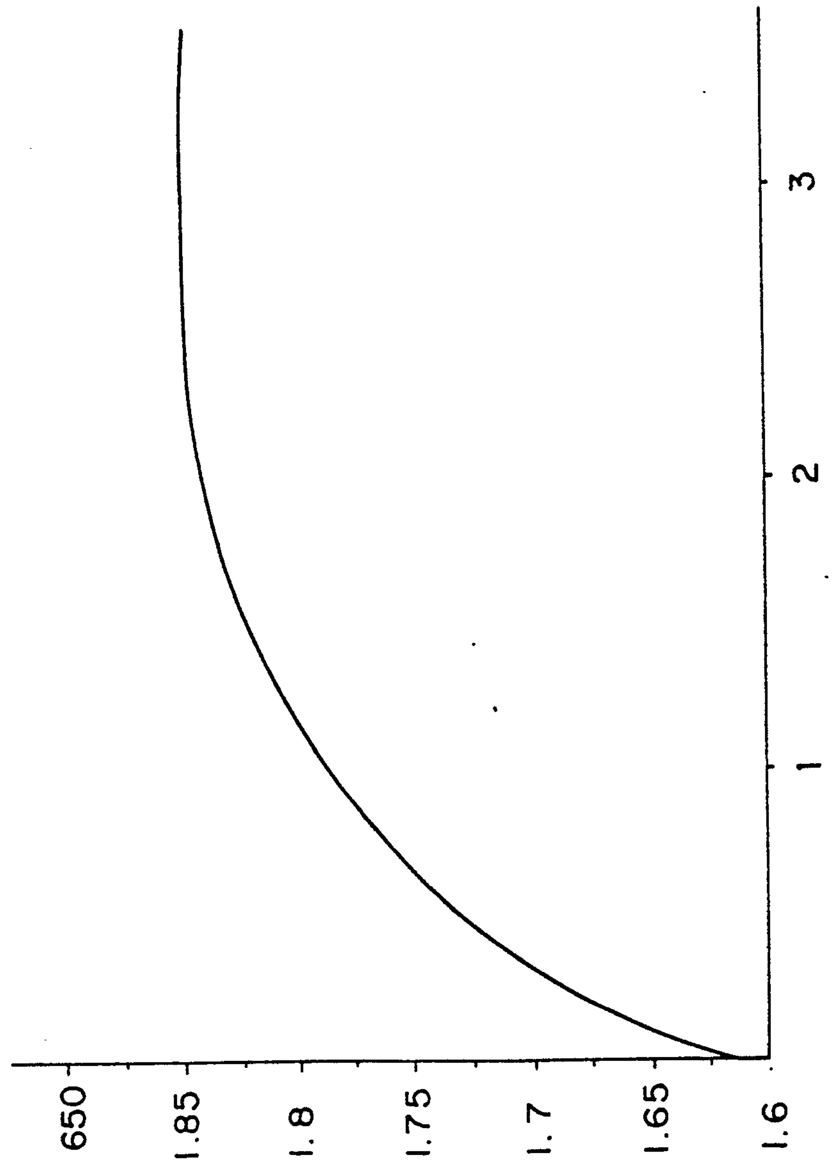

FIG.4

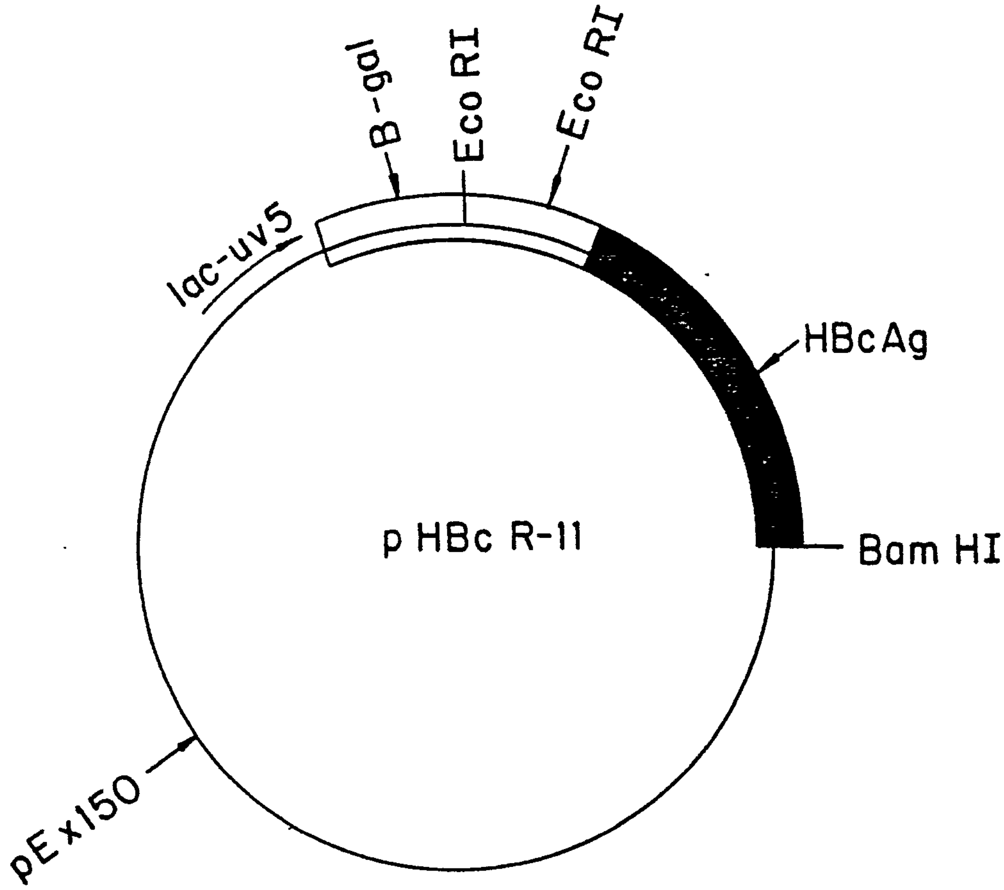

FIG. 5

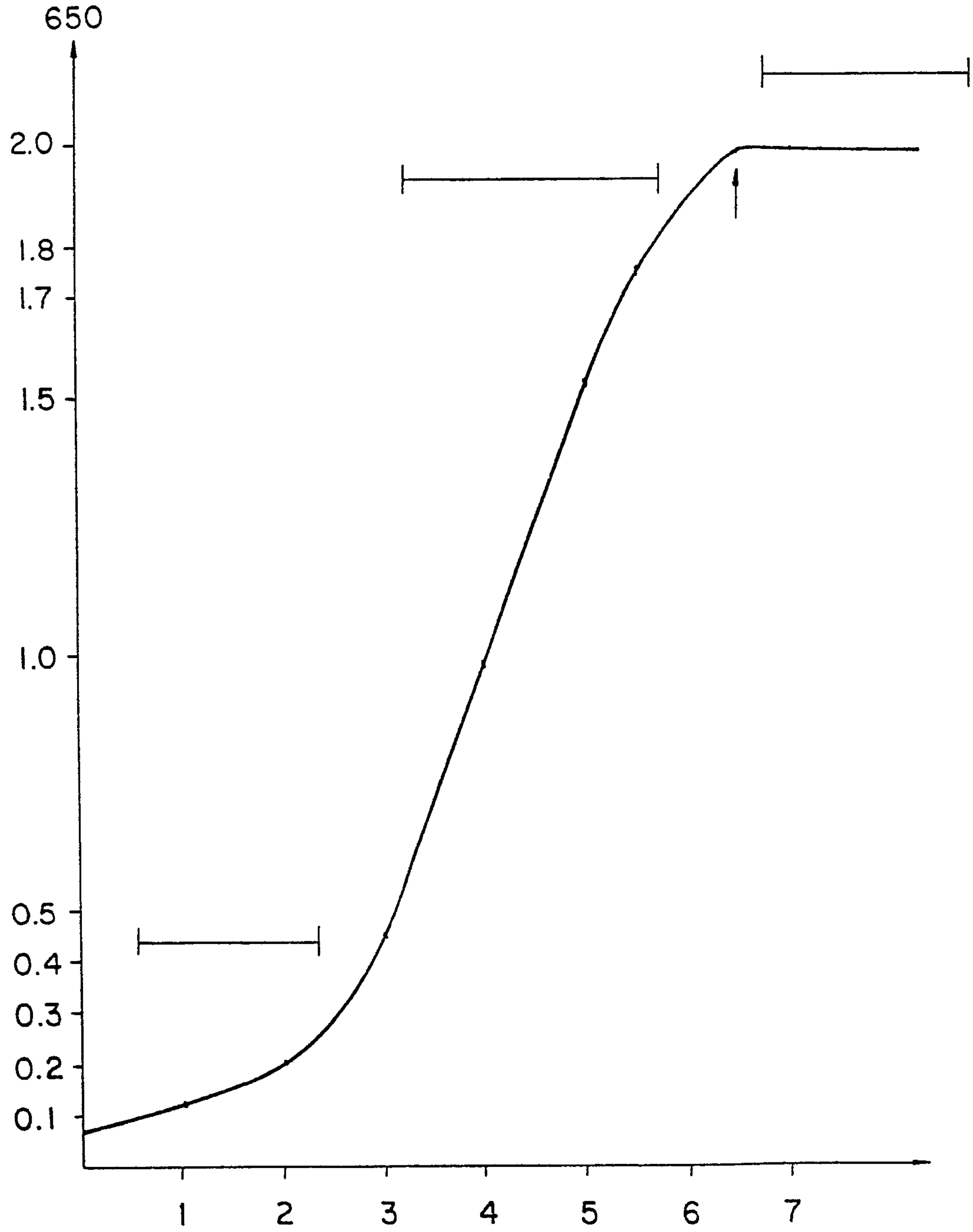

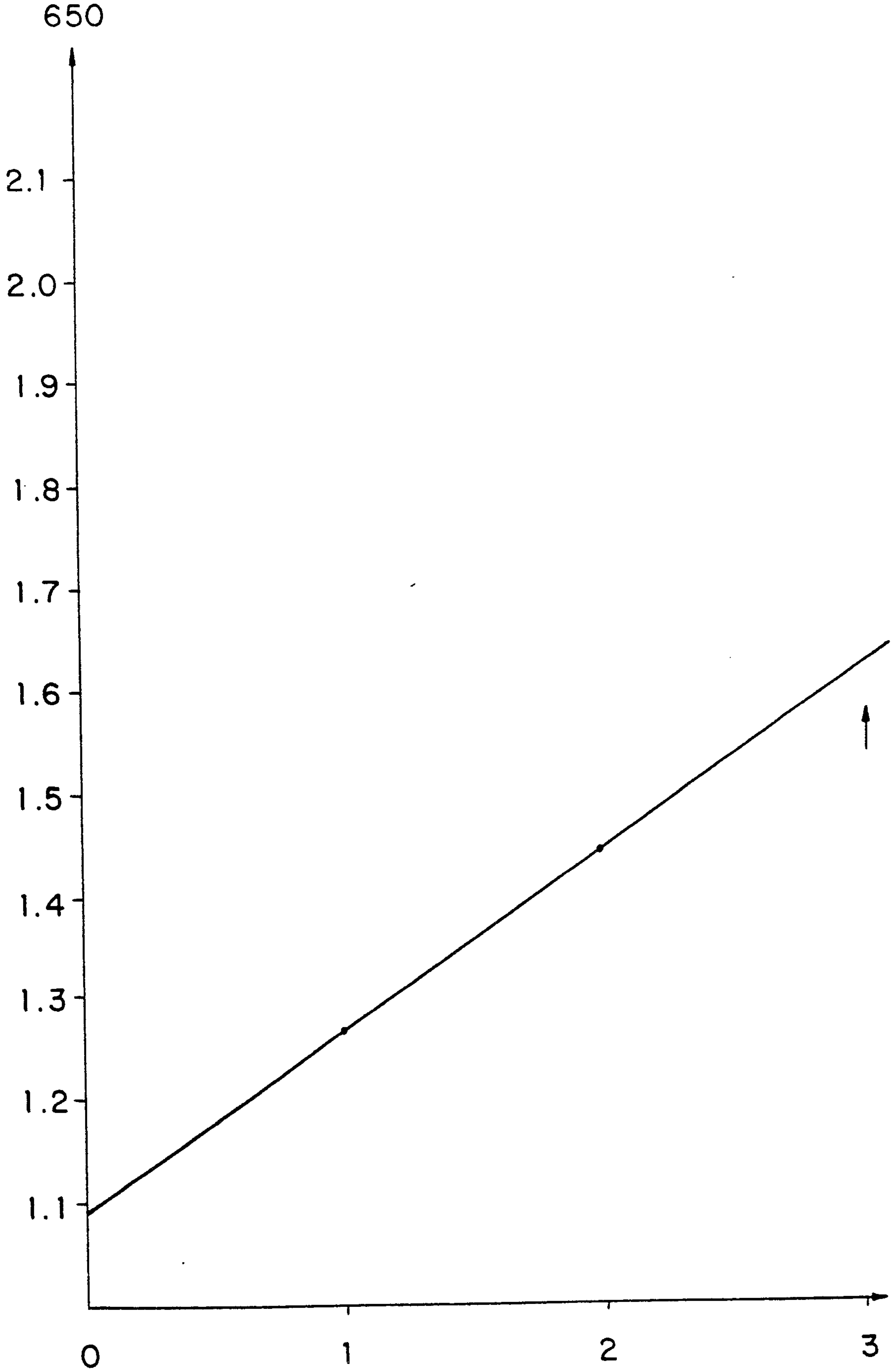
650
2.1
2.0
1.9
1.8
1.7
1.6
1.5
1.4
1.3
1.2
1.1
0
1
2
3